# EUROPEAN PATENT SPECIFICATION

(11) **EP 0 832 131 B1**
(45) Date of publication and mention of the grant of the patent: **16.02.2000**
(21) Application number: 96918239.3
(22) Date of filing: 05.06.1996
(51) Int. Cl.: C07K 14/805

(54) **SEPARATING UNMODIFIED HEMOGLOBIN FROM CROSS-LINKED HEMOGLOBIN**
ABTRENNUNG VON NICHTMODIFIZIERTEM HÄMOGLOBIN AUS VERNETZTEM HÄMOGLOBIN
SEPARATION D'HEMOGLOBINE NON MODIFIEE ET D'HEMOGLOBINE RETICULEE

(30) Priority: 07.06.1995 US 477916
(43) Date of publication of application: 01.04.1998
(73) Proprietor: BIOPURE CORPORATION, Cambridge, MA 02141 (US)
(72) Inventor: LIGHT, William, R., Natick, MA 01760 (US); GAWRYL, Maria, S., Charlestown, MA 02129 (US); LACCETTI, Anthony, J., North Andover, MA 01845 (US); HOUTCHENS, Robert, A., Milford, MA 01757 (US)
(74) Representative: Holdcroft, James Gerald, Dr.
(86) International application number: PCT/US96/09251
(87) International publication number: WO 96/40783

(56) References cited:
- WO-A-87/07832
- WO-A-88/03408

## Description

### Background of the Invention

There exists a need for a blood-substitute to treat or prevent hypoxia resulting from blood loss (e.g, from acute hemorrhage or during surgical operations), resulting from anemia (e.g., pernicious anemia or sickle cell anemia), or resulting from shock (e.g, volume deficiency shock, anaphylactic shock, septic shock or allergic shock).

The use of blood and blood fractions as in these capacities as a blood-substitute is fraught with disadvantages. For example, the use of whole blood often is accompanied by the risk of transmission of hepatitis-producing viruses and AIDS-producing viruses which can complicate patient recovery or result in patient fatalities. Additionally, the use of whole blood requires blood-typing and cross-matching to avoid immunohematological problems and interdonor incompatibility.

Hemoglobin, as a blood-substitute, possesses osmotic activity and the ability to transport and transfer oxygen. However, aqueous hemoglobin exists in equilibrium between the tetrameric (MW 68,000) and dimeric (MW 34,000) forms. Hemoglobin dimers are excreted by the kidney and result in rapid intravascular elimination of hemoglobin solutions with such solutions typically having a 2-4 hour plasma half-life.

Efforts have been directed to overcome the inherent limitations of hemoglobin solutions by molecularly modifying the hemoglobin. Intramolecularly and intermolecularly cross-linking of hemoglobin has generally reduced renal elimination and increased intravascular retention time.

However, solutions of cross-linked hemoglobin still typically contain a significant fraction of unmodified tetrameric hemoglobin. This unmodified tetrameric hemoglobin can convert to dimeric hemoglobin and then be excreted from the body, thereby reducing the average intravascular retention time for cross-linked hemoglobin blood-substitutes. Furthermore, current means for separation, such as standard filtration, do not adequately distinguish between unmodified tetrameric hemoglobin and modified tetrameric hemoglobin.

Thus, in spite of the recent advances in the preparation of cross-linked hemoglobin blood-substitutes, the need continues to exist for a method to effectively separate unmodified hemoglobin from a solution of an intramolecularly and/or intermolecularly cross-linked hemoglobin blood-substitute to improve the average intravascular retention time of the blood-substitute and to prevent significant levels of renal excretion of hemoglobin (see WO-A-87/07832 and WO-A-88/03408).

### Summary of the Invention

The present invention relates to a method for separating unmodified hemoglobin from cross-linked hemoglobin in a hemoglobin solution. The method involves contacting the hemoglobin solution with a least one dissociating agent to form a dissociation solution wherein unmodified tetrameric hemoglobin is dissociated to form hemoglobin dimers. The hemoglobin dimers are then separated from the dissociation solution, while retaining the cross-linked hemoglobin in the dissociation solution.

The advantages of this invention include providing a blood-substitute with an improved intravascular retention time, a reduction or elimination of significant renal elimination of hemoglobin and the side effects associated therewith, a suitable oncotic pressure, and reduced hypertensive effects.

### Brief Description of the Drawings

Figure 1 represents a schematic flow diagram of a method for separating unmodified hemoglobin from modified hemoglobin blood-substitute according to the present invention.

Figure 2 represents the molecular weight distribution of a modified hemoglobin polymer, in solution, treated according to the method of this invention.

### Detailed Description of the Invention

Hemoglobin (Hb) suitable for Hb solutions of this invention can be derived from new, old or outdated blood from humans and other vertebrates, such as cattle, pigs, sheep and chickens. In addition, transgenically-produced hemoglobin, such as the transgenically-produced Hb described in BIO/TECHNOLOGY, 12: 55-59 (1994), and recombinantly produced hemoglobin, such as the recombinantly produced hemoglobin described in Nature, 356: 258-260 (1992), are also suitable for Hb solutions of this invention.

The blood can be collected from live or freshly slaughtered donors. Examples of suitable methods for obtaining hemoglobin, derived from red blood cells, are described in U.S. Patent Nos. 5,084,558 and 5,296,465, issued to Rausch et al.

Hemoglobin is derived from red blood cells, or recombinant bacteria, by a suitable method of washing and lysis, such as are known in the art.

Suitable hemoglobin solutions comprise aqueous solutions of dissolved Hb wherein the dissolved Hb includes unmodified Hb in addition to modified tetrameric Hb and/or polymerized Hb.

Unmodified hemoglobin, as defined herein, is hemoglobin in an non-dissociated tetrameric which can dissociate in aqueous solution into Hb dimers, and dissociated Hb dimers. Hb dimers can further dissociated into Hb subunits (monomers). Unmodified Hb may be free (not polymerized) within a Hb solution and/or may be intermolecularly cross-linked into a polymer chain within the Hb solution.

Cross-linked hemoglobin, as defined herein, is which is modified and/or polymerized. For unmodified Hb contained in a Hb polymer chain, any dimers which are not intermolecularly cross-linked can be dissociated and separated by the method of invention.

Modified hemoglobin, as defined herein, is Hb which has been intramolecularly cross-linked to preclude significant dissociation, in aqueous solution, of Hb tetramers into Hb dimers.

In polymerized hemoglobin, Hb tetramers are intermolecularly cross-linked to form a Hb polymer chain. A hemoglobin polymer can contain modified hemoglobin, unmodified hemoglobin, or a combination thereof. In this method, Hb dimers can be dissociated from unmodified Hb tetramers within a polymer chain, in aqueous solution, if the Hb dimer is not intermolecularly bound to other Hb tetramers.

In the method of the present invention, at least one dissociation agent is contacted with hemoglobin in an aqueous solution to form a dissociation solution. Suitable dissociation agents are water-soluble agents at a concentration within an aqueous solution which, when exposed to unmodified hemoglobin tetramers, result in breaking at least a portion of the hydrogen bonds between Hb dimers in the unmodified Hb tetramers to dissociate the unmodified Hb tetramers into independent α₁β₁ and/or α₂β₂ Hb dimers. The Hb dimers may also further dissociate to form Hb subunits (α₁, α₂, β₁ and β₂).

A dissociation solution typically contains a concentration of dissolved dissociation agent having a normality of about 1 gm-equivalent of dissociation agent per liter of dissociation solution, or more. Preferably, a the concentration of dissociation agent within a dissociation solution is greater than about 1.4 N.

Dissociation agents must be ionic or strongly polar when in an aqueous solution. Examples of suitable dissociation agents include, water soluble inorganic salts (e.g., salts of sodium, calcium, magnesium and zinc), water soluble organic salts (e.g., triethylamine chloride), and water soluble organic amines (e.g., guanidine). Preferably, a dissociation agent is an inorganic salt, or salts, containing at least one multivalent metal cation such as Ca⁺², Mg⁺² or Zn⁺².

Water soluble, as defined herein, means that the material is sufficiently soluble in water at room temperature to form a solution with a concentration sufficient, when contacted with Hb, to result in breaking at least a portion of the hydrogen bonds between Hb dimers in unmodified Hb tetramers.

The dissociation agent can be dissolved in an aqueous solution prior to being contacted with the Hb solution or conversely, the dissociation agent can be a solid, in powder or particulate form, when contacted with an aqueous Hb solution wherein the dissociation agent will then dissolve.

Either the dissociation agent or the Hb solution can be added to the other, or they can be added together. The dissociation agent and the Hb solution are mixed under conditions of relatively low shear.

Examples of suitable hemoglobin solutions include hemoglobin solutions which have a stabilized 2,3-diphosphoglycerate level, as described in U.S. Patent No. 3,864,478, issued to Bonhard; cross-linked hemoglobin, as described in U.S. Patent No. 3,925,344, issued to Mazur, or in U.S. Patent Nos. 4,001,200, 4,001,401 and 4,053,590, issued to Bonsen *et al.,* or in U.S. Patent No. 4,061,736, issued to Morris *et al*., or in U.S. Patent No. 4,473,496, issued to Scannon; stroma-free hemoglobin, as described in U.S. Patent No. 3,991,181, issued to Doczi, or in U.S. Patent No. 4,401,652, issued to Simmonds *et al*. or in U.S. Patent No. 4,526,715, issued to Kothe *et al.;* hemoglobin coupled with a polysaccharide, as described in U.S. Patent No. 4,064,118, issued to Wong; hemoglobin condensed with pyridoxal phosphate, as described in U.S. Patent No. 4,136,093, issued to Bonhard *et al*.; dialdehyde-coupled hemoglobin, as described in U.S. Patent No. 4,336,248, issued to Bonhard *et al.;* hemoglobin covalently bound with inulin, as described in U.S. Patent No. 4,377,512, issued to Ajisaka *et al.;* hemoglobin or a hemoglobin derivative which is coupled with a polyalkylene glycol or a polyalkylene oxide, as described in U.S. Patent No. 4,412,989, issued to Iwashita *et al.,* or U.S. Patent No. 4,670,417, issued to Iwasaki *et al*., or U.S. Patent No. 5,234,903, issued to Nho *et al.*; pyrogen- and stroma-free hemoglobin solution, as described in U.S. Patent No. 4,439,357, issued to Bonhard *et al.;* stroma-free, non-heme protein-free hemoglobin, as described in U.S. Patent No. 4,473,494, issued to Tye; modified cross-linked stroma-free hemoglobin, as described in U.S. Patent No. 4,529,719, issued to Tye; stroma-free, cross-linked hemoglobin, as described in U.S. Patent No. 4,584,130, issued to Bucci *et al.;* α-cross-linked hemoglobin, as described in U.S. Patent Nos. 4,598,064 and Re. 34,271, issued to Walder *et al.*; stable aldehyde polymerized hemoglobin, as described in U.S. Patent No. 4,857,636, issued to Hsia; hemoglobin covalently linked to sulfated glycosaminoglycans, as described in U.S. Patent No. 4,920,194, issued to Feller *et al.;* modified hemoglobin reacted with a high molecular weight polymer having reactive aldehyde constituents, as described in U.S. Patent No. 4,900,780, issued to Cerny; hemoglobin cross-linked in the presence of sodium tripolyphosphate, as described in U.S. Patent No. 5,128,452, issued to Hai *et al.*; stable, polyaldehyde polymerized hemoglobin, as described in U.S. Patent No. 5,189,146, issued to Hsia; and β-cross-linked hemoglobin, as described in U.S. Patent No. 5,250,665, issued to Kluger *et al.* other examples of suitable Hb solutions are described in U.S. 5,296,465, issued to Rausch et al.

In a preferred embodiment, hemoglobin used in the method of invention is in the form of a polymerized hemoglobin blood-substitute. Examples of suitable polymerized hemoglobin blood-substitutes are described in U.S. Patent Nos. 5,084,558 and 5,217,648, issued to Rausch *et al*.

The composition of Hb blood-substitutes preferred for use in the method of invention are sterile aqueous solutions having less than 0.5 endotoxin units/ml, a methemoglobin content that will not result in a significant reduction in oxygen transport/transfer capacity, a total hemoglobin concentration between about 1 to about 25 g Hb/dl, a physiologic pH and a chloride ion concentration of less than 35 meq/l.

The term "endotoxin" refers to the cell-bound lipopolysaccharides produced as a part of the outer layer of bacterial cell walls, which under many conditions are toxic. An endotoxin unit (EU) has been defined, by the United States Pharmacopeial Convention of 1983, page 3013, as the activity contained in 0.1 nanograms of U.S. reference standard lot EC-5. One vial of EC-5 contains 10,000 EU.

Conditions within the dissociation solution, such as pH and temperature, are within ranges which will not significantly reduce the ability of the Hb to transport and release oxygen, such as would occur from denaturing the Hb. Such suitable conditions are as classically known in the art.

The pH of the dissociation solution must be low enough for Hb tetramer dissociation to occur and high enough to preclude significant acid-induced denaturing of the Hb. Typically, pH is maintained between about 4.5 and about 9.5. Preferably, the pH of the dissociation solution is acidic.

In another embodiment, the dissociation solution also contains a buffer to maintain the dissociation solution within a suitable pH range, typically about 4.5 to about 9.5. The buffer can consist of one or more chemical compound.

A preferred buffer comprises 2,2-bis(hydroxymethyl)-2,2',2"-nitrilotriethanol (Bis-Tris) with a pH between 5.5 and 8.0.

The dissociation solution can be buffered by adding a solid (powder or particulate) buffer or an aqueous buffer solution to the Hb solution. Further, the buffer can be added to an aqueous solution of the dissociation agent prior to being contacted with the Hb solution.

In yet another embodiment, the dissociation solution further contains a stabilizing agent in an amount suitable to minimize the formation of methemoglobin by auto-oxidation. An example of a suitable amount cf a stabilizing agent is a 0.1 mM solution of ethylenediaminetetraacetic acid (EDTA).

Hemoglobin solutions used in this method are typically maintained under conditions sufficient to minimize microbial growth, or bioburden, such as maintaining temperature at less than about 20 °C and above 0 °C. Preferably, temperature is maintained at a temperature of about 15 °C or less. More preferably, the temperature is maintained at about 10 °C to about 12 °C.

The dissociation solution is then filtered to purify the Hb solution by separating dissociated Hb dimer from modified Hb and/or polymerized Hb. Suitable filters include ultrafilters which will pass in the filtrate components having a molecular weight cutout between about 40,000 Daltons and 100,000 Daltons. During filtration, components of the Hb solution, which are smaller in diameter than modified tetrameric Hb, or which are fluids or dissolved, pass through the filter with the filtrate. However, the modified Hb tetramers and the polymerized Hb generally remains in the retentate.

A 50,000 Dalton ultrafilter is preferred as it will allow separation of Hb dimers from the Hb solution without a significant loss of yield of modified Hb tetramers or polymerized Hb.

In one embodiment, the dissociation solution is only filtered once. Alternately, the dissociation solution can purified by one then one filter in series, wherein the retentate of from a previous filter is further purified by a subsequent filter.

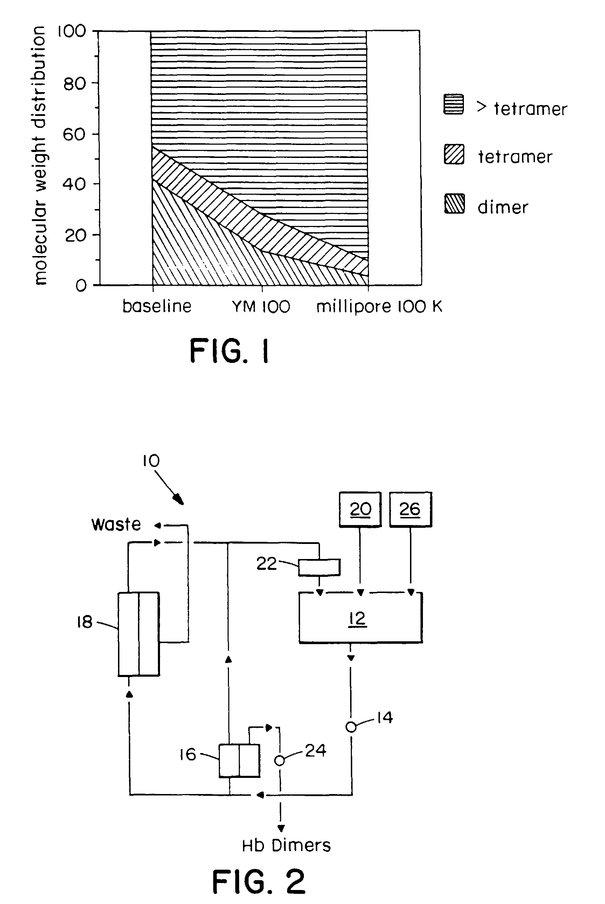

Preferably, the Hb retentate is recirculated continuously through one or more filters, as shown in Figure 1, thereby continuing to remove dimer as unmodified Hb continues to dissociate in the dissociation solution over time.

In another embodiment, water or an aqueous solution of electrolytes, or preferably of dissociation agent, is added to the dissociation solution before and/or during filtration to at least particularly make up for the fluid volume lost as filtrate during filtration. The water or aqueous solution can be added batchwise or continuously at a rate equal to the rate of filtrate volume loss through the filter.

Water as used in the method of invention may be distilled water, deionized water, water-for-injection (WFI) and/or low pyrogen water (LPW). WFI, which is preferred, is deionized, distilled water that meets U.S. Pharmacological Specifications for water-for-injection. WFI is further described in *Pharmaceutical Engineering,* 11, 15-23 (1991). LPW, which is more preferred, is deionized water containing less than 0.002 EU/ml.

Typically, about 99%, or more, of the unmodified Hb has been separated from the modified Hb and polymeric Hb in the Hb retentate, when the volume of filtrate removed from the Hb solution equals about 500% of the volume of the Hb solution prior to adding the dissociation agent.

When using the Hb solution as a blood-substitute, the hemoglobin in the Hb retentate is then washed and equilibrated by diafiltration with a physiologic buffer to ensure the physiological acceptability of the blood-substitute. Suitable physiologic buffers include buffers that have physiologically acceptable levels of electrolytes (e.g, NaCl, KCl and CaCl₂) in WFI. Preferably, the buffer is depyrogenated, such as by filtration with a 10,000 Dalton ultrafilter, and deoxygenated.

A buffer solution can further include a dissolved, non-toxic reducing agent, such as N-acetyl-L-cysteine, cysteine, sodium dithionite or ascorbate, to chemically scavenge oxygen in the blood-substitute to reduce methemoglobin formation. For Hb blood-substitutes, a methemoglobin content of about 25% or more will typically result in a significant reduction in oxygen delivery capacity. It is preferred that methemoglobin content be less than about 15%. In an even more preferred, the methemoglobin content in a Hb blood-substitute be less than or equal to about 10%.

Oxygenation of Hb, similar to dissociation buffers, will also dissociate unmodified hemoglobin into Hb dimers, as shown in Example III. However, oxygenation of Hb also promotes methemoglobin formation.

It is understood that the physiologic buffer and the reducing agent can be added separately, or jointly, to the Hb retentate in a batch or continuous feed mode.

In a preferred embodiment, the Hb retentate is washed by diafilteration against a physiologic buffer until the Hb solution is physiologically acceptable to humans and/or other vertebrates.

Typically, diafiltration continues until the volume of fluid lost through diafiltration across the diafilter is about five times, or more, of the initial volume of the Hb retentate before washing. In a more preferred embodiment diafiltration is continued until about 10 volumes of fluid have been exchanged.

Further description of the use of this method, to remove unmodified Hb from polymeric Hb solutions, is provided in Examples I and II.

In this method, portions of the components for the process for a preparing a stable polymerized hemoglobin blood-substitute are sufficiently sanitized to produce a sterile product. Sterile is as defined in the art, specifically, that the solution meets United States Pharmacopeia requirements for sterility provided in *USP* XXII, Section 71, pages 1483-1488. Further, portions of components that are exposed to the process stream, are usually fabricated or clad with a material that will not react with or contaminate the process stream. Such materials can include stainless steel and other steel alloys, such as Inconel.

The pump used in this method can be a peristaltic-type, diaphragm-type, gear-type, piston-type or rotary-lobe type pump. Diaphragm-type pumps are available from Branne Lubbem Inc., Buffalo Grove, IL. Suitable rotary-lobe pumps include the Albin SLP 110 P51 B1 sanitary lobe-rotary pump from Albin Pump Inc., Atlanta, GA. Rotary-lobe pumps can also be obtained from Waukesha Pumps, Waukesha, WS.

One embodiment of a system 10, suitable for practicing the method of invention for separating unmodified hemoglobin from a Hb solution contained modified Hb tetramer and/or polymeric Hb, is illustrated in Figure I. System 10 includes tank 12, pump 14, purification filter 16 and diafilter 18. Pump 14 takes a suction on tank 12 and recirculates Hb solution through purification filter 16 and/or diafilter 18. It is understood that purification filter 16 and diafilter 18 can be operated in parallel or in series arrangements. Tank 12 contains Hb solution which can be formed within tank 12 or which can be formed prior to being transferred into tank 12.

An amount of an Hb dissociation agent, suitable to dissociate Hb tetrameric molecules into Hb dimers, is then contacted with the Hb solution in system 10 to form a dissociation solution. The dissociation agent is typically introduced into tank 12, from dissociation agent supply 20. However, it is understood that the dissociation agent can be added at other locations in system 10. It is also understood that the dissociation agent can be added to the Hb solution in a batch or continuous feed mode.

The dissociation agent and the Hb solution in the dissociation solution are then mixed by a low shear mixing, specifically static mixer 22. The dissociation agent and the Hb solution are mixed by recirculating the dissociation solution from tank 12, by pump 14 through an orifice, not shown, and static mixer 22.

Static mixer 22 is typically located downstream of pump 14 and upstream of purification filter 16, however, static mixer 22 alternately could be located at other points in system 10.

The unmodified hemoglobin in the dissociation solution then commences to dissociate from unmodified Hb tetramers into Hb dimers. The Hb dimers each have a molecular weight of about 32,000 Daltons.

The dissociation solution is then recirculated through purification filter 16 to remove Hb dimers in the filtrate, and retain modified Hb and polymeric Hb in the Hb retentate. During filtration, components of the dissociation solution, which are smaller in diameter than stabilized tetrameric Hb, or which are fluids, pass through purification filter 16 with the filtrate. Examples of suitable purification filters include ultrafilters with a molecular weight cutout between about 40,000 Daltons and about 100,000 Daltons.

In a continuous feed mode, a liquid or dissolved dissociation agent is added continuously, as makeup, at a rate equal to the rate of filtrate loss through purification filter 16. In another embodiment, the volume of filtrate discharged through purification filter 16 is regulated by filtrate pump 24.

Separation of unmodified Hb from the Hb retentate is typically complete when the volume of filtrate drained from purification filter 16 equals about 500% of the volume of Hb solution contained in tank 22 prior to adding dissociation agent to system 10.

In another embodiment, the Hb retentate is then washed and equilibrated by diafiltration with a physiologic buffer to make the Hb retentate physiologically acceptable as a blood-substitute. A physiologic buffer is introduced into tank 12, from physiologic buffer supply 26. However, it is understood that the physiologic buffer can be added at any location in system 10. It is also understood that the physiologic buffer can be added to the Hb retentate in a batch or continuous feed mode.

A preferred physiologic buffer includes 27 mM sodium lactate, 12 mM N-acetyl-L-cysteine, 115 mM NaCl and 1.36 mM CaCl₂ in WFI (pH 8).

The Hb retentate is then diafiltered by recirculating the Hb retentate and physiological buffer from tank 12, by pump 14 through static mixer 22 and diafilter 18. Diafilter 18 is located downstream of static mixer 22 and upstream of tank 12. Diafiltration continues until the blood-substitute is physiologically acceptable. Typically, the blood-substitute is physiologically acceptable when the volume of fluid lost through diafiltration across diafilter 18 is at least five times the initial volume of the Hb retentate in system 10.

During Hb dissociation and Hb retentate diafiltration, the Hb temperature is maintained at approximately 8 °C to 12 °C in tank 14. An example of an acceptable means for controlling the Hb temperature is by cooling the outside of tank 14 through use of an ethylene glycol jacketed cooling system, not shown.

The invention will be further illustrated by the following non-limiting examples.

### Example I

### Diafiltration of Deoxygenated Hb Solution Containing a Higher Concentration Dissociation Buffer

A polymerized Hb solution was formed according to the method described in Example 1 of U.S. Patent No. 5,084,558, issued to Rausch *et al*. This Hb solution was analyzed by gel permeation chromatography (GPC) and found to comprise about 45% Hb dimers, about 15% unmodified Hb tetramers, and about 40% polymerized Hb molecules which were larger than unmodified tetramers.

One liter of a dissociation buffer containing 1.5 M MgCl₂, 0.1 M Bis-Tris and 0.2 mM EDTA (pH 6.5) was added to one liter of the Hb solution. This mixture was then recirculated through a 100 kD polysulfone ultrafilter (Millipore Catalog No. PTHK 000C5) to concentrate the mixture to a volume of one liter. The concentrated mixture was subsequently diafiltered with 11 volumes of a dissociation buffer comprising 0.7 M MgCl₂, 0.05 M Bis-Tris and 0.1 mM EDTA (pH 6.5). The filtered Hb solution was then washed and equilibrated with a deoxygenated buffer containing 27 mM sodium lactate, 12 mM N-acetyl cysteine, 115 mM NaCl, 4 mM KCl, and 1.36 mM CaCl₂ in WFI. The molecular weight distribution of the resulting Hb solution was then analyzed by GPC.

The results of these analyses are shown in Figure II. The Hb solution was then found to have a final composition of about 5% Hb dimers, about 10% Hb tetramers and about 85% polymerized Hb molecules which were larger than tetramers.

### Example II

### Diafiltration of Deoxygenated Hb Solution Containing a Lower Concentration Dissociation Buffer

One hundred seventy mLs of a dissociation buffer containing 0.75 M MgCl₂, 0.05 M Bis-Tris and 0.1 mM EDTA (pH 7.5) was added to 15 mLs of the initial polymerized Hb solution of Example I and then 15 mLs of a two-fold concentrate of the dissociation buffer was added. The Hb solution was then recirculated through a Chemineer, Inc./Kenics static mixer and then diafiltered by a 100 kD ultrafilter (Amicon YM 100, Catalog No. 14451) to obtain 200 mls of Hb solution.

The Hb solution was then diafiltered with 3 volume exchanges of the dissociation buffer and lastly washed and equilibrated with a deoxygenated buffer containing 27 mM sodium lactate, 12 mM N-acetyl cysteine, 115 mM NaCl, 4 mM KCl, and 1.36 mM CaCl₂ in WFI. The molecular weight distribution of the resulting Hb solution was then analyzed by GPC.

The results of these analyses are shown in Figure II. The Hb solution was then found to have a final composition of about 15% Hb dimers, about 15% Hb tetramers and about 70% polymerized Hb molecules which were larger than tetramers. Thus, the method of invention was effective in reducing the content of unmodified hemoglobin in the polymerized Hb solutions.

### Example III

### Synthesis and Diafiltration of Oxygenated and Deoxygenated Hb Solutions Without a Dissociation Buffer

As described in U.S. Patent No. 5,296,465, samples of bovine whole blood were collected, mixed with a sodium citrate anticoagulant to form a blood solution, and then analyzed for endotoxin levels.

Each blood solution sample was maintained after collection at a temperature of about 2 °C and then strained to remove large aggregates and particles with a 600 mesh screen.

Prior to pooling, the penicillin level in each blood solution sample was assayed with an assay kit purchased from Difco, Detroit, Michigan using the method entitled "Rapid Detection of Penicillin in Milk" to ensure that penicillin levels in the blood solutions were ≤ 0.008 units/ml.

The blood solution samples were then pooled and mixed with depyrogenated aqueous sodium citrate solution to form a 0.2% by weight solution of sodium citrate in bovine whole blood (hereafter "0.2% sodium citrate blood solution").

The 0.2% sodium citrate blood solution was then passed, in-series, through 800 µm and 50 µm polypropylene filters to remove large blood solution debris of a diameter approximately 50 µm or more.

The RBCs were then washed to separate extracellular plasma proteins, such as BSA or IgG, from the RBCs. To wash the RBCs contained in the blood solution, the volume of blood solution in the diafiltration tank was initially diluted by the addition of an equal volume of a filtered isotonic solution to diafiltration tank. The isotonic solution was filtered with a Millipore (Cat # CDUF 050 G1) 10,000 Dalton ultrafiltration membrane. The isotonic solution was composed of 6.0 g/l sodium citrate dihydrate and 8.0 g/l sodium chloride in water-for-injection (WFI). The diluted blood solution was then concentrated back to its original volume by diafiltration through a 0.2 µm hollow fiber (Microgon Krosflo II microfiltration cartridge) diafilter. Concurrently, filtered isotonic solution was added continuously, as makeup, at a rate equal to the rate of filtrate loss through the 0.2 µm diafilter. During diafiltration, components of the diluted blood solution which were significantly smaller in diameter than RBCs, or are fluids such as plasma, passed through the walls of the 0.2 µm diafilter with the filtrate. RBCs, platelets and larger bodies of the diluted blood solution, such as white blood cells, were retained with continuously-added isotonic solution to form a dialyzed blood solution.

During RBC washing, the diluted blood solution was maintained at a temperature between approximately 10 to 25 °C with a fluid pressure at the inlet of the diafilter between about 120 N/m² (25 psi) and about 140 N/m² (30 psi) to improve process efficiency.

RBC washing was complete when the volume of filtrate drained from the diafilter equaled about 600% of the volume of blood solution prior to diluting with filtered isotonic solution.

The dialyzed blood solution was then continuously pumped at a rate of approximately 4 lpm to a Sharples Super Centrifuge, Model # AS-16, fitted with a #28 ringdam. The centrifuge was operating while concurrently being fed dialyzed blood solution, to separate the RBCs from the white blood cells and platelets. During operation, the centrifuge rotated at a rate sufficient to separate the RBCs into a heavy RBC phase, while also separating a substantial portion of the white blood cells (WBCs) and platelets into a light WBC phase, specifically about 15,000 rpm. A fraction or the RBC phase and of the WBC phase were separately and continuously discharged from the centrifuge during operation.

Following separation of the RBCs, the RBCs were lysed to form a hemoglobin-containing solution. A substantial portion of the RBCs were mechanically lysed while discharging the RBCs from the centrifuge. The cell membranes of the RBCs ruptured upon impacting the wall of RBC phase discharge line at an angle to the flew of RBC phase out of the centrifuge, thereby releasing hemoglobin (FBI from the RBCs into the RBC phase.

The lysed RBC phase then flowed through the RBC phase discharge line into a static mixer (Kenics 1/2 inch with 6 elements, Chemineer, Inc.). Concurrent with the transfer of the RBC phase to the static mixer, an equal amount of WFI was also injected into the static mixer, wherein the WFI mixed with the RBC phase. The flow rates of the RBC phase and the WFI into the static mixer are each at about 4x10⁻⁶ m³/sec (0.25 lpm).

Mixing the RBC phase with WFI in the static mixer produced a lysed RBC colloid. The lysed RBC colloid was then transferred from the static mixer into a Sharples Super Centrifuge (Model # AS-16, Sharples Division of Alfa-Laval Separation, Inc.) which was suitable to separate the Hb from non-hemoglobin RBC components. The centrifuge was rotated at a rate sufficient to separate the lysed RBC colloid into a light Hb phase and a heavy phase. The light phase was composed of Hb and also contained non-hemoglobin components with a density approximately equal to or less than the density of Hb.

The Hb phase was continuously discharged from the centrifuge, through a 0.45 µm Millipore Pellicon Cassette, Cat # HVLP 000 C5 microfilter, and into a holding tank in preparation for Hb purification. Cell stroma were then returned with the retentate from the microfilter to the holding tank. During microfiltration, the temperature within the holding tank was maintained at 10 °C or less. To improve efficiency, when the fluid pressure at the microfilter inlet increased from an initial pressure of about 48 N/m² (10 psi) to about 120 N/m² (25 psi), microfiltration was complete. The Hb microfiltrate was then transferred from the microfilter into the microfiltrate tank.

Subsequently, the Hb microfiltrate was pumped through a 100,000 Millipore Cat # CDUF 050 H1 ultrafilter. A substantial portion of the Hb and water, contained in the Hb microfiltrate, permeated the 100,000 Dalton ultrafilter to form a Hb ultrafiltrate, while larger cell debris, such as proteins with a molecular weight above about 100,000 Dalton, were retained and recirculated back into the microfiltrate tank. Concurrently, WFI was continuously added to the microfiltrate tank as makeup for water lost in the ultrafiltrate. Generally, cell debris include all whole and fragmented cellular components with the exception of Hb, smaller cell proteins, electrolytes, coenzymes and organic metabolic intermediates. Ultrafiltration continued until the concentration of Hb in the microfiltrate tank was less than 8 grams/liter (g/l). While ultrafiltering the Hb, the internal temperature of the microfiltrate tank was maintained at about 10°C.

The Hb ultrafiltrate was transferred into an ultrafiltrate tank, wherein the Hb ultrafiltrate was then recirculated through a 30,000 Dalton Millipore Cat # CDUF 050 T1 ultrafilter to remove smaller cell components, such as electrolytes, coenzymes, metabolic intermediates and proteins less than about 30,000 Daltons in molecular weight, and water from the Hb ultrafiltrate, thereby forming a concentrated Hb solution containing about 100 g Hb/l.

The concentrated Hb solution was then directed from the ultrafiltrate tank onto the media contained in parallel chromatographic columns (2 feet long with an 8 inch inner diameter) to separate the Hb by high performance liquid chromatography. The chromatographic columns contained an anion exchange medium suitable to separate Hb from non-hemoglobin proteins. The anion exchange media was formed from silica gel. The silica gel was exposed to γ-glycidoxy propylsilane to form active epoxide groups and then exposed to C₃H₇(CH₃)NCl to form a quaternary ammonium anion exchange medium. This method of treating silica gel is described in the *Journal of Chromatography,* 120:321-333 (1976).

Each column was pre-treated by flushing the chromatographic columns with a first buffer which facilitated Hb binding. Then 4.52 liters of the concentrated Hb solution were injected into each chromatographic column. After injecting the concentrated Hb solution, the chromatographic columns were then washed by successively directing three different buffers through the chromatographic columns to produce a Hb eluate, by producing a pH gradient within the columns. The temperature of each buffer during use was about 12.4 °C. The buffers were prefiltered through 10,000 Dalton ultrafiltration membrane before injection onto the chromatographic columns.

The first buffer, 20 mM tris-hydroxymethyl aminomethane (Tris) (pH about 8.4 to about 9.4), transported the concentrated Hb solution into the media in the chromatographic columns to bind the Hb. The second buffer, a mixture of the first buffer and a third buffer, with the second buffer having a pH of about 8.3, then adjusted the pH within chromatographic columns to elute contaminating non-hemoglobin components from the chromatographic columns, while retaining the Hb. Equilibration with the second buffer continued for about 30 minutes at a flow rate of approximately 5.93x10⁻⁵ m³/sec (3.56 lpm) per column. The elute from the second buffer was discarded to waste. The third buffer, 50 mM Tris (pH about 6.5 to about 7.5), then eluted the Hb from the chromatographic columns.

The Hb eluate was then directed through a 0.22 µ Sartobran Cat # 5232507 G1PH filter to a tank wherein the Hb eluate was collected. The first 3-to-4% of the Hb eluate and the last 3-to-4% of the Hb eluate were directed to waste.

The Hb eluate was further used if the eluate contained less than 0.05 EU/ml of endotoxin and contained less than 3.3 nmoles/ml phospholipids. To sixty liters of ultrapure eluate, which had a concentration of 100 g Hb/l, was added 9 l of 1.0 M NaCl, 20 mM Tris (pH 8.9) buffer, thereby forming an Hb solution with an ionic strength of 160 mM, to reduce the oxygen affinity of the Hb in the Hb solution. The Hb solution was then concentrated at 10 °C, by recirculating through the ultrafilter, specifically a 10,000 Dalton Millipore Helicon, Cat # CDUF050G1 filter, until the Hb concentration was 110 g/l.

The Hb solution was then deoxygenated, until the pO₂ of the Hb solution was reduced to the level where HbO₂ content was about 10%, by recirculating the Hb solution at 12 lpm, through a 0.05 µm Hoechst-Celanese Corporation Cat # G-240/40) polypropylene microfilter phase transfer membrane, to form a deoxygenated Hb solution (hereinafter "deoxy-Hb"). Concurrently, a 60 lpm flow of nitrogen gas was directed through the counter side of the phase transfer membrane. During deoxygenation, the temperature of the Hb solution was maintained between about 19 °C and about 31 °C.

Also during deoxygenation, and subsequently throughout the process, the Hb was maintained in a low oxygen environment to minimize oxygen absorption by the Hb and to maintain an oxygenated Hb (oxyhemoglobin or HbO₂) content of less than about 10% in the deoxy-Hb.

The deoxy-Hb, 60 l, was then diafiltered through an ultrafilter with 180 l of a storage buffer, containing 0.2 wt % N-acetyl cysteine, 33 mM sodium phosphate buffer (pH 7.8) having a pO₂ of less than 6.7x10³ N/m² (50 torr), to form a oxidation-stabilized deoxy-Hb. Prior to mixing with the deoxy-Hb, the storage buffer was depyrogenated with a 10,000 Dalton Millipore Helicon, Cat # CDUF050G1 depyrogenating filter.

The storage buffer was continuously added at a rate approximately equivalent to the fluid loss across the ultrafilter. Diafiltration continued until the volume of fluid lost through diafiltration across the ultrafilter was about three times the initial volume of the deoxy-Hb. The material may be stored at this point.

Prior to transferring the oxidation-stabilized deoxy-Hb into a polymerization apparatus, oxygen-depleted WFI was added to the polymerization reactor to purge the polymerization apparatus of oxygen to prevent oxygenation of oxidation-stabilized deoxy-Hb. The amount of WFI added to the polymerization apparatus was that amount which would result in a Hb solution with a concentration of about 40 g Hb/l, when the oxidation-stabilized deoxy-Hb was added to the polymerization reactor. The WFI was then recirculated throughout the polymerization apparatus, to deoxygenate the WFI by flow through a 0.05 µm polypropylene microfilter phase transfer membrane (Hoechst-Celanese Corporation Cat # 5PCM-108, 7.4 m² (80 sq. ft.)) against a counterflow of pressurized nitrogen. The flow rates of WFI and nitrogen gas, through the phase transfer membrane, were about 3x10⁻⁴ to 3.3x10⁻⁴ m³/sec (18 to 20 lpm) and 6.7x10⁻⁴ to 1x10⁻³ m³/sec (40 to 60 lpm), respectively.

After the p0₂ of the WFI in polymerization apparatus was reduced to less than about 2 torr pO₂, the polymerization reactor was blanketed with nitrogen by a flow of about 3.33x10⁻⁴ m³/sec (20 lpm) of nitrogen into the head space of the polymerization reactor. The oxidation-stabilized deoxy-Hb was then transferred into the polymerization reactor.

The polymerization was conducted in a 12 nM phosphate buffer with a pH of 7.8, having a chloride concentration less than or equal to about 35 mmolar which was produced by mixing the Hb solution with WFI.

The oxidation-stabilized deoxy-Hb and N-acetyl cysteine were subsequently slowly mixed with the cross-linking agent glutaraldehyde, specifically 29.4 grams of glutaraldehyde for each kilogram of Hb over a five hour period, while heating at 42 °C and recirculating the Hb solution through a Kenics 1-1/2 inch static mixer with 6 elements (Chemineer, Inc.), to form a polymerized Hb (poly(Hb)) solution.

Recirculating the oxidation-stabilized deoxy-Hb and the glutaraldehyde through the static mixer caused turbulent flow conditions with generally uniform mixing of the glutaraldehyde with the oxidation-stabilized deoxy-Hb, thereby reducing the potential for forming pockets of deoxy-Hb containing high concentrations of glutaraldehyde. Generally uniform mixing of glutaraldehyde and deoxy-Hb reduced the formation of high molecular weight poly(Hb) (having a molecular weight above 500,000 Daltons) and also permitted faster mixing of glutaraldehyde and deoxy-Hb during polymerization.

In addition, significant Hb intramolecular cross-linking resulted during Hb polymerization as an effect of the presence of N-acetyl cysteine upon the polymerization of Hb.

After polymerization, the temperature of the poly(Hb) solution in the polymerization reactor was reduced to a temperature between about 15 °C to about 25 °C.

The poly(Hb) solution was then concentrated by recirculating the poly(Hb) solution through the ultrafilter until the concentration of the poly(Hb) was increased to about 85 g/l. A suitable ultrafilter is a 30,000 Dalton filter (e.g., Millipore Helicon, Cat # CDUF050LT).

Subsequently, the poly(Hb) solution was then mixed with 66.75 g sodium borohydride, and then again recirculated through the static mixer. Specifically, for every nine liters of poly(Hb) solution, one liter of 0.25 M sodium borohydride solution was added at a rate of 0.1 to 0.12 lpm.

Prior to adding the sodium borohydride to the poly(Hb) solution, the pH of the poly(Hb) solution was basified by adjusting pH to a pH of about 10 to preserve the sodium borohydride and to prevent hydrogen gas formation. The pH of the poly(Hb) solution was adjusted by diafiltering the poly(Hb) solution with approximately 215 l of depyrogenated, deoxygenated 12 mM sodium borate buffer, having a pH of about 10.4 to about 10.6. The poly(Hb) solution was diafiltered by recirculating the poly(Hb) solution from the polymerization reactor through the 30 kD ultrafilter. The sodium borate buffer was added to the poly(Hb) solution at a rate approximately equivalent to the rate of fluid loss across the ultrafilter from diafiltration. Diafiltration continued until the volume of fluid lost across the ultrafilter from diafiltration was about three times the initial volume of the poly(Hb) solution in the polymerization reactor.

Following pH adjustment, sodium borohydride solution was added to the polymerization reactor to reduce imine bonds in the poly(Hb) solution to ketimine bonds and to form stable poly(Hb) in solution. During the sodium borohydride addition, the poly(Hb) solution in the polymerization reactor was continuously recirculated through the static mixer and the 0.05 µm polypropylene microfilter phase transfer membrane to remove dissolved oxygen and hydrogen. Flow through a static mixer also provided turbulent sodium borohydride flow conditions that rapidly and effectively mixed sodium borohydride with the poly(Hb) solution. The flow rates of poly(Hb) solution and nitrogen gas through the 0.05 µm phase transfer membrane were between about 3.3x10⁻⁵ to 6.7x10⁻⁵ m³/sec (2.0 to 4.0 lpm) and about 2x10⁻⁴ to 3x10⁻⁴ m³/sec (12 to 18 lpm), respectively. After completion of the sodium borohydride addition, reduction continued in the polymerization reactor while an agitator contained therein rotated at approximately 75 rotations per minute.

Approximately one hour after the sodium borohydride addition, the stable poly(Hb) solution was recirculated from the polymerization reactor through the 30,000 Dalton ultrafilter until the stable poly(Hb) solution concentration was 110 g/l. Following concentration, the pH and electrolytes of the stable poly(Hb) solution were restored to physiologic levels to form a stable polymerized Hb blood-substitute, by diafiltering the stable poly(Hb) solution, through the 10,000 Dalton ultrafilter, with a filtered, deoxygenated, low pH buffer containing 27 mM sodium lactate, 12 mM NAC, 115 mM NaCl, 4 mM KCl, and 1.36 mM CaCl₂ in WFI, (pH 5.0). Diafiltration continued until the volume of fluid lost through diafiltration across the ultrafilter was about 6 times the pre-diafiltration volume of the concentrated Hb product.

After the pH and electrolytes were restored to physiologic levels, the stable polymerized Hb blood-substitute was then diluted to a concentration of 5.0 g/dl by adding the filtered, deoxygenated low pH buffer to polymerization reactor. The diluted blood-substitute was then diafiltered by recirculating from the polymerization reactor through the static mixer and a 100,000 Dalton purification filter against a filtered deoxygenated buffer containing 27 mM sodium lactate, 12 mM NAC, 115 mM NaCl, 4 mM KCl, and 1.36 mM CaCl₂ in WFI, (pH 7.8). Diafiltration continued until the blood-substitute contained less than or equal to about 10% modified tetrameric and unmodified tetrameric species by GPC when run under dissociating conditions.

The purification filter was run under conditions of low transmembrane pressure with a restricted permeate line. Following removal of substantial amounts of modified tetrameric Hb and unmodified tetrameric Hb, recirculation of the blood-substitute continued through the 30,000 Dalton ultrafilter until the concentration of the blood-substitute was about 130 g/l.

The stable blood-substitute was then stored in a suitable container having a low oxygen environment and a low oxygen in-leakage.

This Hb solution was subsequently analyzed by GPC and found to comprise about 3.5% Hb dimers, 31% unmodified Hb tetramers and about 65.5% polymerized Hb molecules which were larger than unmodified tetramers.

Two liters of the Hb solution were oxygenated through an oxygenation cartridge with a gaseous mixture, comprising 98% oxygen and 2% carbon dioxide, until 95% oxygenated Hb valves were obtained by a co-oximeter (Co-Oximeter Model #482; Instrumentation Laboratory, Lexington, MA).

The oxygenated Hb solution was then diafiltered with 7 volumes of an oxygenated buffer solution containing 27 mM solution lactate, 12 mM N-acetyl-L-cysteine, 115 mM NaCl, 4 mM KCl and 1.4 mM CaCl₂ in WFI against a 100 kD ultrafilter. Throughout this process, the Hb solution was not contacted with any dissociation buffer.

The molecular weight distribution of the resulting Hb solution was then analyzed by GPC. The molecular weight distribution was found to be 0.5% dimer and 2.7% unmodified tetramer and about 96.8% polymerized Hb molecules which were larger than unmodified tetramers.

This entire procedure was then repeated on another sample of the same polymerized Hb solution, with the exception that the Hb solution was not oxygenated prior to diafiltration. The molecular weight distribution of the resulting Hb solution was found by GPC to be 2.2% dimer, 2.5% unmodified tetramer and about 95.3% polymerized Hb molecules which were larger than unmodified tetramers.

The results of these procedures show that oxygenation of hemoglobin likewise promotes the dissociation of unmodified Hb tetramer to Hb dimers. However, oxygenation also promotes the formation of the undesirable methemoglobin.

### Example IV

### Molecular Weight Analysis

Molecular weight was determined by conducting gel permeation chromatography (GPC) on the hemoglobin solutions under dissociating conditions. This method of analysis results in the separation of hemoglobin polymers on the basis of size, with larger molecules eluting faster than smaller molecules. By comparison to protein molecular weight standards, it is possible for correlate elution time with the molecular weights of the hemoglobin products.

In this analysis, a representative sample of the hemoglobin product was analyzed for molecular weight distribution. The hemoglobin product was diluted to 4 mg/mL within a mobile phase of 50 mM Bis-Tris (pH 6.5), 750 mM MgCl₂, and 0.1 mM EDTA. The diluted sample was injected onto a HPLC TosoHaas G3000SW column. Flow rate is 0.5 ml/min. and ultraviolet detection was set at @280 nm.

## Claims

1. A method for separating unmodified hemoglobin from cross-linked hemoglobin in a hemoglobin solution, comprising the steps of:
a) contacting the hemoglobin solution with a least one dissociating agent to form a dissociation solution wherein unmodified tetrameric hemoglobin is dissociated to form hemoglobin dimers; and
b) separating the hemoglobin dimers from the dissociation solution, while retaining the cross-linked hemoglobin in said dissociation solution.

2. A method of Claim 1 wherein at least a portion of the hemoglobin in the hemoglobin solution is polymerized.

3. A method of Claim 1 or Claim 2 wherein the dissociation agent is a water soluble inorganic salt.

4. A method of Claim 3 wherein the inorganic salt (a) is a sodium salt; or (b) includes a multivalent cation, for example selected from Ca⁺², Mg⁺² Zn⁺² and combinations thereof.

5. A method of Claim 1 or Claim 2 wherein the dissociation agent is a water soluble organic salt, e.g. salt of an amine.

6. A method of Claim 1 or Claim 2 wherein the dissociation agent is a water soluble organic amine, e.g. guanidine.

7. A method of Claim 1 or Claim 2 further comprising the step of contacting the hemoglobin solution with a buffer, e.g. 2,2-bis(hydroxy-methyl)-2,2',2"-nitrilotriethanol.

8. A method of Claim 1 or Claim 2 further comprising the step of contacting the hemoglobin solution with a stabilizing agent, e.g. ethylenediaminetetraacetic acid.

9. A method of any one of the preceding claims wherein the unmodified hemoglobin is separated from the dissociation solution by filtering the dissociation solution.

10. A method of Claim 1 or Claim 2 further comprising the step of washing the cross-linked hemoglobin with a physiologically acceptable buffer, after separating the unmodified hemoglobin from the dissociation solution, to produce a physiologically acceptable hemoglobin blood-substitute.

11. A method for separating unmodified hemoglobin from cross-linked in a hemoglobin solution to produce a physiologically acceptable blood-substitute, comprising the steps of:
a) contacting the hemoglobin solution with a dissociating agent to form a dissociation solution, wherein unmodified tetrameric hemoglobin dissociated to form hemoglobin dimers;
b) filtering the hemoglobin dimers from the dissociation solution while retaining cross-linked hemoglobin in the retentate; and
c) washing the cross-linked hemoglobin with a physiologic buffer to produce a physiologically acceptable blood-substitute.

12. A method of Claim 11 wherein the buffer contains sodium lactate and N-acetyl-L-cysteine.

13. A method for separating unmodified hemoglobin free cross-linked hemoglobin in a hemoglobin solution, comprising the steps of:
a) mixing the hemoglobin solution with a dissociation agent, a buffer and a stabilizing agent to form a dissociation solution, wherein the unmodified hemoglobin dissociates to form hemoglobin dimers within the dissociation solution;
b) filtering the dissociation solution to separate the hemoglobin dimers from the cross-linked hemoglobin; and
e) washing the cross-linked hemoglobin solution with a deoxygenated solution containing sodium lactate, N-acetyl-L-cysteine and physiologic electrolytes.

14. A blood substitute comprising a hemoglobin product produced or producible by a method according to anyone of the preceding claims.

15. A blood substitute comprising a product according to Claim 14 for use in therapy.

16. Use of a product obtainable by a method of any of the preceding claims 1-13 for the manufacture of a blood substitute for in vivo use.

## Patentansprüche

1. Ein Verfahren zur Trennung nichtmodifizierten Hämoglobins von vernetztem Hämoglobin in einer Hämoglobin-Lösung, wobei das Verfahren die Schritte umfasst:
a) In-Kontakt-Bringen der Hämoglobin-Lösung mit mindestens einem Dissoziierungsmittel, um eine Dissoziationslösung zu bilden, worin nichtmodifiziertes tetrameres Hämoglobin unter Bildung von Hämoglobin-Dimeren dissoziiert ist; und
b) Trennen der Hämoglobin-Dimeren aus der Dissoziationslösung unter Zurückhalten des vernetzten Hämoglobins in besagter Dissoziations lösung.

2. Ein Verfahren nach Anspruch 1, worin mindestens ein Teil des Hämoglobins in der Hämoglobin-Lösung polymerisiert ist.

3. Ein Verfahren nach Anspruch 1 oder Anspruch 2, worin das Dissoziationsmittel ein wasserlösliches anorganisches Salz ist.

4. Ein Verfahren nach Anspruch 3, worin das anorganische Salz (a) ein Natriumsalz ist; oder (b) ein mehrwertiges Kation einschließt, das zum Beispiel aus Ca²⁺, Mg²⁺, Zn²⁺ und Kombinationen davon ausgewählt ist.

5. Ein Verfahren nach Anspruch 1 oder Anspruch 2, worin das Dissoziationsmittel ein wasserlösliches organisches Salz, z.B. Salz eines Amins, ist.

6. Ein Verfahren nach Anspruch 1 oder Anspruch 2, worin das Dissoziationsmittel ein wasserlösliches organisches Amin, z.B. Guanidin, ist.

7. Ein Verfahren nach Anspruch 1 oder Anspruch 2, das außerdem den Schritt des In-Kontakt-Bringens der Hämoglobin-Lösung mit einem Puffer, z.B. 2,2-Bis(hydroxy-methyl)-2,2',2"-nitrilotriethanol, umfasst.

8. Ein Verfahren nach Anspruch 1 oder Anspruch 2, das außerdem den Schritt des In-Kontakt-Bringens der Hämoglobin-Lösung mit einem Stabilisierungsmittel, z.B. Ethylendiamintetraessigsäure, umfasst.

9. Ein Verfahren nach einem der vorausgehenden Ansprüche, worin das nichtmodifizierte Hämoglobin von der Dissoziationslösung durch Filtrieren der Dissoziationslösung getrennt wird.

10. Ein Verfahren nach Anspruch 1 oder Anspruch 2, das außerdem den Schritt des Waschens des vernetzten Hämoglobins mit einem physiologisch geeigneten Puffer nach Trennen des nichtmodifizierten Hämoglobins von der Dissoziationslösung umfasst, zur Herstellung eines physiologisch verträglichen Hämoglobin-Blutersatzmittels.

11. Ein Verfahren zur Trennung nichtmodifizierten Hämoglobins von vernetztem Hämoglobin in einer Hämoglobin-Lösung, zur Herstellung eines physiologisch verträglichen Blutersatzmittels, wobei das Verfahren die Schritte umfasst:
a) In-Kontakt-Bringen der Hämoglobin-Lösung mit einem Dissoziierungsmittel, zur Bildung einer Dissoziationslösung, worin nichtmodifiziertes tetrameres Hämoglobin zur Bildung von Hämoglobin-Dimeren dissoziiert ist;
b) Filtrieren der Hämoglobin-Dimeren von der Dissoziationslösung, unter Zurückhalten vernetzten Hämoglobins im Rückstand; und
c) Waschen des vernetzten Hämoglobins mit einem physiologischen Puffer zur Herstellung eines physiologisch verträglichen Blutersatzmittels.

12. Ein Verfahren nach Anspruch 11, worin der Puffer Natriumlactat und N-Acetyl-L-cystein enthält.

13. Ein Verfahren zur Trennung nichtmodifizierten Hämoglobins von vernetztem Hämoglobin in einer Hämoglobin-Lösung, wobei das Verfahren die Schritte umfasst:
a) Mischen der Hämoglobin-Lösung mit einem Dissoziationsmittel, einem Puffer und einem Stabilisierungsmittel zur Bildung einer Dissoziationslösung, worin das nichtmodifizierte Hämoglobin dissoziiert, zur Bildung von Hämoglobin-Dimeren in der Dissoziationslösung;
b) Filtrieren der Dissoziationslösung zur Trennung der Hämoglobin-Dimeren von dem vernetzten Hämoglobin; und
e) Waschen der Lösung des vernetzten Hämoglobins mit einer von Sauerstoff befreiten Lösung, die Natriumlactat, N-Acetyl-L-cystein und physiologische Elektrolyte enthält.

14. Ein Blutersatzmittel, das ein Hämoglobin-Produkt umfaßt, das mit einem Verfahren gemäß einem der vorausgehenden Ansprüche hergestellt oder herstellbar ist.

15. Ein Blutersatzmittel, das ein Produkt gemäß Anspruch 14 zur therapeutischen Anwendung umfasst.

16. Verwendung eines Produkts, das durch ein Verfahren nach einem der vorausgehenden Ansprüche 1 bis 13 erhältlich ist, zur Herstellung eines Blutersatzmittels zur in vivo Anwendung.

## Revendications

1. Procédé de séparation d'hémoglobine non modifiée et d'hémoglobine réticulée dans une solution d'hémoglobine, comprenant les étapes consistant à :
a) mettre en contact la solution d'hémoglobine avec au moins un agent de dissociation de façon à former une solution de dissociation dans laquelle l'hémoglobine tétramère non modifiée est dissociée de façon à former des dimères d'hémoglobine ; et
b) séparer les dimères d'hémoglobine de la solution de dissociation tout en retenant l'hémoglobine réticulée au sein de ladite solution de dissociation.

2. Procédé selon la revendication 1 caractérisé en ce qu'au moins une partie de l'hémoglobine de la solution d'hémoglobine est polymérisée.

3. Procédé selon la revendication 1 ou la revendication 2 caractérisé en ce que l'agent de dissociation est un sel inorganique soluble dans l'eau.

4. Procédé selon la revendication 3 caractérisé en ce que le sel inorganique :
(a) est un sel de sodium ; ou
(b) comprend un cation multivalent, par exemple choisi parmi Ca²⁺, Mg²⁺, Zn²⁺ et leurs combinaisons.

5. Procédé selon la revendication 1 ou la revendication 2 caractérisé en ce que l'agent de dissociation est un sel organique soluble dans l'eau, notamment un sel correspondant à une amine.

6. Procédé selon la revendication 1 ou la revendication 2 caractérisé en ce que l'agent de dissociation est une amine organique soluble dans l'eau, notamment la guanidine.

7. Procédé selon la revendication 1 ou la revendication 2 comprenant en outre une étape consistant à mettre en contact la solution d'hémoglobine avec un tampon, notamment le 2,2-bis(hydroxyméthyl)-2,2',2"-nitrilotriéthanol.

8. Procédé selon la revendication 1 ou la revendication 2 comprenant en outre une étape consistant à mettre en contact la solution d'hémoglobine avec un agent stabilisateur, notamment l'acide éthylènediaminetétraacétique.

9. Procédé selon l'une quelconque des revendications caractérisé en ce que l'hémoglobine non modifiée est séparée de la solution de dissociation par filtrage de la solution de dissociation.

10. Procédé selon la revendication 1 ou la revendication 2 comprenant en outre une étape consistant à laver l'hémoglobine réticulée avec un tampon physiologiquement acceptable, après séparation de l'hémoglobine non modifiée de la solution de dissociation, de façon à produire un substitut sanguin à base d'hémoglobine et physiologiquement acceptable.

11. Procédé de séparation d'hémoglobine non modifiée et d'hémoglobine réticulée dans une solution d'hémoglobine, de façon à produire un substitut sanguin physiologiquement acceptable, et comprenant les étapes consistant à :
a) mettre en contact la solution d'hémoglobine avec un agent de dissociation de façon à former une solution de dissociation dans laquelle l'hémoglobine tétramère non modifiée est dissociée de façon à former des dimères d'hémoglobine ;
b) filtrer les dimères d'hémoglobine de la solution de dissociation tout en retenant l'hémoglobine réticulée dans le retentat ; et
c) laver l'hémoglobine réticulée avec un tampon physiologiquement acceptable de façon à produire un substitut sanguin physiologiquement acceptable.

12. Procédé selon la revendication 11 caractérisé en ce que le tampon contient du lactate de sodium et de la N-acétyl-L-cystéine.

13. Procédé de séparation d'hémoglobine non modifiée et d'hémoglobine réticulée dans une solution d'hémoglobine, comprenant les étapes consistant à :
a) mélanger la solution d'hémoglobine avec un agent de dissociation, un tampon et un agent stabilisateur de façon à former une solution de dissociation, dans laquelle l'hémoglobine non modifiée se dissocie de façon à former des dimères d'hémoglobine de la solution de dissociation ;
b) filtrer la solution de dissociation de façon à séparer les dimères d'hémoglobine de l'hémoglobine réticulée ; et
c) laver la solution d'hémoglobine réticulée avec une solution désoxygénée contenant du lactate de sodium, de la N-acétyl-L-cystéine et des électrolytes physiologiques.

14. Substitut sanguin comprenant un produit à base d'hémoglobine produit ou productible par un procédé selon l'une quelconque des revendications précédentes.

15. Substitut sanguin comprenant un produit à base d'hémoglobine selon la revendication 14 pour une utilisation en thérapie.

16. Utilisation d'un produit pouvant être obtenu par un procédé selon l'une quelconque des revendications précédentes 1 à 13 pour la fabrication d'un substitut sanguin pour utilisation in vivo.
